# EUROPÄISCHE PATENTSCHRIFT

(11) **EP 0 553 365 B1**
(45) Veröffentlichungstag und Bekanntmachung des Hinweises auf die Patenterteilung: **21.09.1994**
(21) Anmeldenummer: 92101211.8
(22) Anmeldetag: 25.01.1992
(51) Int. Cl.: A44C 7/00, A61H 39/04

(54) **Ohrschmuck**
Earring
Boucle d'oreille

(43) Veröffentlichungstag der Anmeldung: 04.08.1993
(73) Patentinhaber: Hieber, Fritz E.W. Dr. med., D-76530 Baden-Baden (DE)
(72) Erfinder: Hieber, Fritz E.W., Dr. Med., W-7570 Baden-Baden (DE); Lang, Dieter, W-7130 Mühlacker (DE); Pfeffer, Gudrun, W-7532 Niefern (DE)
(74) Vertreter: Trappenberg, Hans

(56) Entgegenhaltungen:
- PATENT ABSTRACTS OF JAPAN vol. 3, no. 108 (M-072)29. Juni 1979 & JP-A-54 081 978(Hitachi Metals Ltd.)
- PATENT ABSTRACTS OF JAPAN vol. 3, no. 108 (M-072)3. Juli 1979 & JP-A-54 083 584

## Beschreibung

Die Erfindung betrifft Ohrschmuck zur Stimulation von an der Ohrmuschel vorhandenen Akupunkturpunkten beziehungsweise -linien (Siehe auch JP-A-54 083 584), gebildet durch ein etwa kreisringförmig gebogenes offenes Schmuckstück beliebiger Form, dessen eine Stirnseite zum Einhängen in die Ohrmuschelgrube bestimmt ist, während sich das andere Ende an der Ohrmuschelhinterwand abstützt, wobei das Schmuckstück aus elektrischen Strom leitendem Material ist und die Stirnflächen aus Materialien gebildet sind, die entsprechend der elektrochemischen Spannungsreihe ein gegenseitiges Potential aufweisen.

Bei derartigem Ohrschmuck entsteht durch die an den Stirnflächen des Ohrschmucks vorhandenen unterschiedlichen Materialien, zusammen mit dem Säuremantel der Haut ein galvanisches Element, was geeignet ist durch den steten Stromfluß Akupunkturwirkungen hervorzurufen. Hierbei liegt der eine Endpunkt des Ohrschmucks in der Ohrmuschelgrube fest, während der zweite Endpunkt frei an der Hinterwand der Ohrmuschel wählbar ist. Dadurch können auch Akupunkturpunkte beziehungsweise -linien erreicht werden, die beispielsweise durch übliche Kreolen nicht belegt werden können.

Entsprechend dem Verwendungszweck wurde bisher derartiger Ohrschmuck ausschließlich aus Edelmetall gefertigt, um sowohl einer Reizung der belegten Hautpartien, wie insbesondere auch der Auslösung von Allergien vorzubeugen. Dadurch jedoch wurde derartiger Ohrschmuck schon vom Material her sehr teuer. Hinzu kam noch die aufwendige Herstellung, die insbesonders durch das Einfügen des andersartigen Materials an der einen Stirnfläche bedingt war.

Die Erfindung gibt eine Möglichkeit, derartige Ohrringe zur Stimulierung von Akupunkturpunkten und -linien auf einfachste Weise und daher sehr preiswert herzustellen. Erreicht wird dies in erfindungsgemäßer Weise dadurch, daß der Schmuckkörper aus Gußbronze ist mit einem am einen Ende eingegossenen Endstück aus Eisen.

Nicht also wie bisher, wird der Ohrschmuck aus Edelmetall gefertigt und in schwieriger Arbeit das andersartige Material eingefügt, sondern es wird aus Gußbronze hergestellt, in den ein Eisen-Endstück eingegossen werden kann. Gußbronze, nach der Erfindung eine Zinnbronze mit etwa 15 Vol.% Zinn und mit Zusätzen von Blei und Antimon, ist sehr einfach zu verarbeiten und verbindet sich auch während des Gießvorganges mit dem Eisen-Endstück. Außerdem haben diese beiden Materialien, auf der einen Seite die Zinnbronze, auf der anderen Seite das Eisen, in der elektrochemischen Spannungsreihe einen solchen Abstand voneinander, daß zusammen mit dem Säuremantel der Haut ein Strom fließt, der nach Ansicht von Akupunkturfachleuten in der Größenordnung liegt, um tatsächlich eine Stimulierung der betroffenen Akupunkturpunkte zu erzielen. Diese Wirkung kann noch dadurch verstärkt werden, daß die Stirnflächen mit Vertiefungen versehen sind, in denen sich Schweiß und andere Absonderungen sammeln können, so daß stets das zum Erzielen des Stromflusses notwendige saure Milieu an der Hautoberfläche vorhanden ist. Selbstverständlich kann das Eisen auch mit Legierungszusätzen versehen sein, insbesondere um eine zu starke Korrosion zu vermeiden, jedoch sollte es auf jeden Fall, um keine Allergie hervorzurufen, nickelfrei sein. Bewährt hat es sich, daß das gesamte Schmuckstück hauchvergoldet wird, zum einen um dem Schmuckstück einen besonderen Glanz zu geben, zum anderen aber auch um das Eisen-Endstück während der Lagerung vor Korrosion zu schützen.

Auf der Zeichnung ist ein Ausführungsbeispiel des Erfindungsgegenstandes schematisch dargestellt und zwar zeigen:
Fig. 1 einen kreolenförmigen Ohrschmuck
Fig. 2 eine Ansicht einer Stirnfläche und
Fig. 3 einen Querschnitt bei dem Eisen-Endstück.

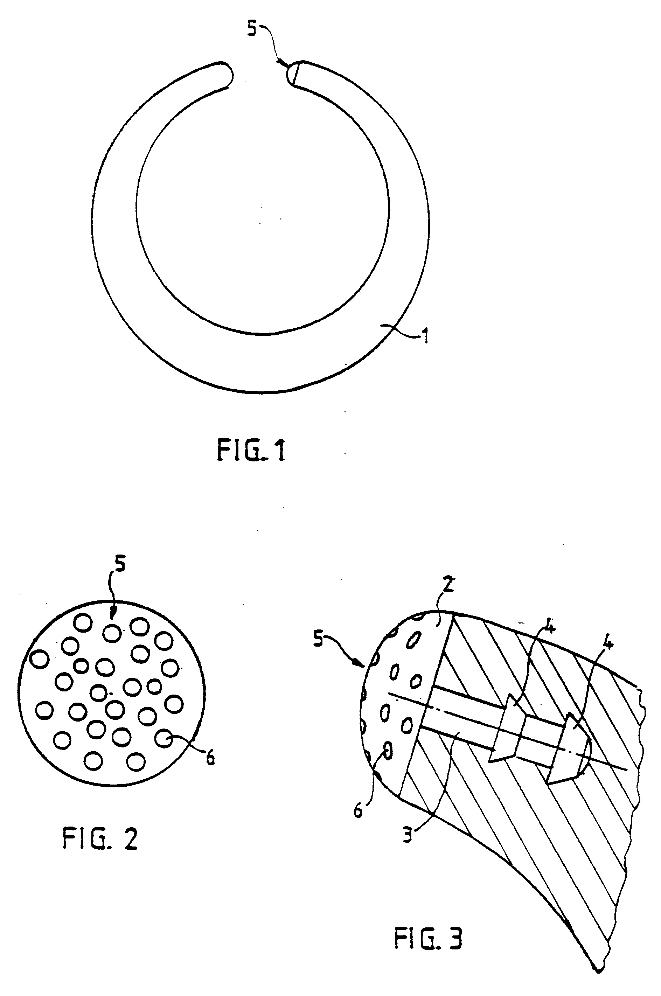

Der Körper des in Fig. 1 dargestellten Ohrschmucks (1) ist aus Gußbronze gegossen, wobei beim Gießvorgang ein pilzförmiges, aus Eisen bestehendes Endstück (2) mit eingegossen wird. Dieses Endstück weist an seinem Stiel (3) Verzahnungskörper (4) auf, um einen sicheren Halt dieses Eisen-Endtückes (2) im Bronzematerial zu erhalten. Die Stirnflächen (5) des Schmuckkörpers (1) sind mit napfförmigen Vertiefungen (6) als Reservoir für flüssige Hautabsonderungen versehen, wodurch sich an den Auflagestellen (5) stets ein saures Milieu ausbildet.

Selbstverständlich kann der aus Gußbronze bestehende Körper des Ohrschmucks (1) noch zusätzlich mit einer Hartbronzeschicht und auch noch mit einer Hauchgoldschicht versehen werden, um zum einen dem Schmuckstück ein ansprechendes Aussehen zu geben und zum anderen es zumindest während der Lagerzeit vor Korrosion zu bewahren.

## Patentansprüche

1. Ohrschmuck zur Stimulation von an der Ohrmuschel vorhandenen Akupunkturpunkten beziehungsweise -linien, gebildet durch ein etwa kreisringförmig gebogenes offenes Schmuckstück beliebiger Form, dessen eine Stirnseite zum Einhängen in die Ohrmuschelgrube bestimmt ist, während sich das andere Ende an der Ohrmuschelhinterwand abstützt, wobei das Schmuckstück aus elektrischen Strom leitenden Material ist und die Stirnflächen aus Materialien gebildet sind, die entsprechend der elektrochemischen Spannungsreihe ein gegenseitiges Potential aufweisen,
dadurch gekennzeichnet,
daß der Schmuckkörper (1) aus Gußbronze ist, mit einem an einem Ende eingegossenen Endstück aus Eisen.

2. Ohrschmuck nach Anspruch 1,
dadurch gekennzeichnet,
daß die Gußbronze eine zinkfreie Zinnbronze mit etwa 15 Vol.% Zinn und mit Zusätzen von Blei und Antimon ist.

3. Ohrschmuck nach Anspruch 1 oder 2,
dadurch gekennzeichnet,
daß das Endstück eine nickelfreie Eisenlegierung ist.

4. Ohrschmuck nach Anspruch 3,
dadurch gekennzeichnet,
daß die Eisenlegierung nichtrostend ist.

5. Ohrschmuck nach einem oder mehreren Ansprüchen,
dadurch gekennzeichnet,
daß die Stirnflächen (5) mit Vertiefungen (6) versehen sind.

6. Ohrschmuck nach einem oder mehreren Ansprüchen,
dadurch gekennzeichnet,
daß das Schmuckstück (1) hauchvergoldet ist.

## Claims

1. An earring for stimulation of acupuncture points or lines present at the pinna, formed by a piece of jewellery of any shape, which is bent approximately in the form of a circular ring and which is open and of which one end is intended for being hung in the pinna hollow while the other end bears against the pinna rear wall, wherein the piece of jewellery is of electrical current-conducting material and the end faces are formed from materials which are of opposite potential in accordance with the electromotive series, characterised in that the body (1) of the jewellery is of cast bronze, with an end portion of iron, which is cast in at one end.

2. An earring according to claim 1 characterised in that the cast bronze is a zinc-free tin bronze with about 15% by volume of tin and with additives of lead and antimony.

3. An earring according to claim 1 or claim 2 characterised in that the end portion is a nickel-free iron alloy.

4. An earring according to claim 3 characterised in that the iron alloy is stainless.

5. An earring according to one or are claims characterised in that the end faces (5) are provided with recesses (6).

6. An earring according to one or are claim characterised in that the piece of jewellery (1) has very fine gold plating thereon.

## Revendications

1. Boucle d'oreille pour stimuler des points ou des lignes d'acuponcture situé(e)s sur le pavillon de l'oreille, constituée par une boucle de forme quelconque, ouverte et courbée approximativement en forme d'anneau, dont l'une des faces frontales est destinée à être accrochée dans le creux du pavillon de l'oreille, tandis que son autre extrémité prend appui sur l'arrière du pavillon de l'oreille, sachant que la boucle est composée d'une matière conductrice de courant électrique et que les surfaces frontales sont constituées par des matières qui, en fonction de la chaîne des forces électrochimiques, présentent des tensions en opposition, caractérisée en ce que le corps de la boucle (1) est en bronze coulé muni d'une partie terminale en fer coulée à une extrémité.

2. Boucle d'oreille selon la revendication 1, caractérisée en ce que le bronze coulé est un bronze ordinaire exempt de zinc contenant approximativement 15 Vol. % d'étain avec addition de plomb et d'antimoine.

3. Boucle d'oreille selon la revendication 1 ou 2, caractérisée en ce que la partie terminale est un alliage de fer exempt de nickel.

4. Boucle d'oreille selon la revendication 3, caractérisée en ce que l'alliage de fer est inoxydable.

5. Boucle d'oreille selon une ou plusieurs revendications, caractérisée en ce que les surfaces frontales (5) sont munies de renfoncements (6).

6. Boucle d'oreille selon une ou plusieurs revendications, caractérisée en ce que le bijou (1) est pour d'un placage d'or.
